# EUROPEAN PATENT APPLICATION

(11) **EP 1 936 380 A1**
(43) Date of publication of application: **25.06.2008**
(21) Application number: 06126879.3
(22) Date of filing: 21.12.2006
(51) Int. Cl.: G01N 33/68, G01N 33/74

(54) **Placental growth factor, soluble FLT1 and endoglin are predictors of the angiogenic status of a subject**

(71) Applicant: F. Hoffmann-La Roche AG, 4070 Basel (CH); Roche Diagnostics GmbH, 68305 Mannheim (DE)
(72) Inventor: Hess, Georg, Prof. Dr., 55130 Mainz (DE); Horsch, Andrea, Dr., 68259 Mannheim (DE); Zdunek, Dietmar, Dr., 82327 Tutzing (DE)
(74) Representative: Dick, Alexander

(57) **Abstract**

The present invention relates to diagnostic means and methods. Specifically, the present invention relates to a method for diagnosing the angiogenic status of a subject suffering from coronary heart disease. Further, it relates to a method of diagnosing whether a subject suffering from coronary heart disease is susceptible for a pro-angiogenic therapy comprising measuring PLGF, Endoglin and sFLT1. Finally, the present invention encompasses diagnostic devices and kits for carrying out the aforementioned methods.

## Description

The present invention relates to diagnostic means and methods. Specifically, the present invention relates to a method for diagnosing the angiogenic status of a subject suffering from coronary heart disease. Further, it relates to a method of diagnosing whether a subject suffering from coronary heart disease is susceptible for a pro-angiogenic therapy. Finally, the present invention encompasses diagnostic devices and kits for carrying out the aforementioned methods.

Angiogenesis is known as the formation of new blood vessels from already existing blood vessels by a capillary sprouting process. The process is under physiological conditions essentially driven by angiogenic growth factors such as the vascular endothelial growth factor (VEGF). The expression of such angiogenic growth factors is regulated pivotally by hypoxia. Thus, if a tissue becomes ischemic, the cells will start to produce angiogenic growth factors which will attract new blood vessels to the tissue by angiogenesis.

However, the capability of a subject for angiogenesis, i.e. its angiogenic status, is dependent on complex biological parameters. Various angiogenesis promoting factors as well as inhibitors of angiogenesis have been reported (Nyberg 2005, Cancer Res 65:3967-3979).

Angiogenesis is observed during tumor growth where the growing tumor becomes more and more affected by hypoxia.

Other disease conditions which are accompanied by hypoxia and ischemia include the coronary heart diseases. Said diseases are characterized by stenosis or occlusion of vessels of the coronary artery system, e.g. by atherosclerosis or thromboembolic occlusions.

Coronary heart diseases result in ischemia of the myocardium. Said ischemia, if left untreated, may severely interfere with the physiological function of the heart and result in cardiac disorders including heart failure or even myocardial infarction. For patients suffering from coronary heart diseases, an angiogenic therapy may assist in avoiding the aforementioned life-threatening conditions. Moreover, angiogeic therapies may even help to circumvent complicated cardiac interventions such as stent implantation or bypass surgery.

As set forth above already, various factors besides VEGF have been reported to play a role in angiogenesis. Placental growth factor (P1GF) is a closely related growth factor suggested to play a role in the related process of arteriogenesis together with its putative receptor Flt-1 (Khurana 2005, Circulation 111:2828-2836). Other factors which are possibly involved in arteriogenesis and angiogenesis are the members of the Transforming growth factor-beta superfamily as well as their receptors or binding partners such as the ALK receptors or Endoglin (van Laake 2006, Circulation, 114:2288-2297; Bobik 2006, Arterioscler Thromb Vasc Biol 26: 1712-1720; Bertolino 2005, Chest Supplement 128: 585-590). Fibroblast growth factor (FGF), Platlet derived growth factor (PDGF) as well as cytokines and matrix-metalloproteinases have been also described as potent angiogenic factors (Nyberg, loc.cit.).

It is to be understood from the above that it is highly desirable to determine the angiogenic status of a subject suffering from coronary heart diseases, specifically in emergency cases. Based on such an assessment of the angiogenic status, it can be predicted whether a subject will be susceptible for a pro-angiogenic therapy in order to prevent the severe outcomes of a coronary heart disease.

Thus, the technical problem underlying the present invention could be seen as the provision of means and methods for determining the angiogenic status of a subject in order to, e.g., select a suitable therapy. The technical problem is solved y the embodiments characterized in the accompanying claims and herein below.

Accordingly, the present invention relates to a method for diagnosing the angiogenic status of a subject suffering from coronary heart disease comprising:
a) determining the amounts of PLGF or a variant thereof, Endoglin or a variant thereof and sFLT1 or a variant thereof in a sample of a subject suffering from coronary heart disease; and
b) comparing the amounts determined in step a) with reference amounts, whereby the angiogenic status is to be diagnosed.

The method of the present invention, preferably, is an in vitro method. Moreover, it may comprise steps in addition to those explicitly mentioned above. For example, further steps may relate to sample pre-treatments or evaluation of the results obtained by the method. The method of the present invention may be also used for monitoring, confirmation, and subclassification of the angiogenic status. The method may be carried out manually or assisted by automation. Preferably, step (a) and/or (b) may in total or in part be assisted by automation, e.g., by a suitable robotic and sensory equipment for the determination in step (a) or a computer-implemented comparison in step (b).

The term "diagnosing" as used herein refers to assessing the probability according to which a subject has a certain angiogenic status, i.e. a pro-angiogenic or an anti-angiogenic status, referred to in this specification. As will be understood by those skilled in the art, such an assessment is usually not intended to be correct for 100% of the subjects to be diagnosed. The term, however, requires that a statistically significant portion of subjects can be correctly diagnosed to exhibit the said angiogenic status. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

The term "angiogenic status" as used herein refers to the capability of a subject of forming blood vessels from already existing blood vessels, e.g., by sprouting. Specifically, it has been found that depending on the amounts of the molecules referred to herein present in a subject, angiogenesis may elicited without further ado, i.e. by the physiological initiators and, preferably, by hypoxia, or may further require exogenously supplied initiators such as angiogenic drugs specified elsewhere in the description. Thus, the angiogentic status, i.e. the capability for angiogenesis in a subject, is determined by the physiological constitution of a subject with respect to the aforementioned molecules.

The term "subject" as used herein relates to animals, preferably mammals, and, more preferably, humans. However, it is envisaged by the present invention that the subject shall be suffering from coronary heart disease as specified elsewhere herein.

The term "coronary heart disease" refers to coronary artery diseases including stenosis, atherosclerosis of the coronary vessels or occlusions, such as thromboembolic occlusions within the coronary vessel system. Preferably, a coronary heart disease as used herein is accompanied by a systolic dysfunction characterized by a left ventricular ejection fraction (LVEF) of less than 60% and, more preferably, less than 40% and at least one stenosis resulting in 50% diminished volume. Moreover, a coronary heart disease, preferably, will appear in coronary angiography as a one, two or three vessel diseases.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The term "P1GF (Placental Growth Factor)" as used herein refers to a placenta derived growth factor which is a 149-amino-acid-long polypeptide and is highly homologous (53% identity) to the platelet-derived growth factor-like region of human vascular endothelial growth factor (VEGF). Like VEGF, P1GF has angiogenic activity in vitro and in vivo. For example, biochemical and functional characterization of P1GF derived from transfected COS-1 cells revealed that it is a glycosylated dimeric secreted protein able to stimulate endothelial cell growth in vitro (Maqlione 1993, Oncogene 8(4):925-31). Preferably, P1GF refers to human P1GF, more preferably, to human P1GF having an amino acid sequence as shown in Genebank accession number P49763, GI: 17380553 (Genebank is available from the NCBI, USA under www.ncbi.nlm.nih.gov/entrez). Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific P1GF. Variants may be allelic variants, splice variants or any other species specific homologs, paralogs, or orthologs. Moreover, the variants referred to herein include fragments of the specific P1GF or the aforementioned types of variants as long as these fragments have the essential immunological and biological properties as referred to above. Such fragments may be, e.g., degradation products of P1GF. Further included are variants which differ due to posttranslational modifications such as phosphorylation or myristylation.

The term "Endoglin" as used herein refers to a polypeptide having a molecular weight of 180 kDa non-reduced, 95 kDa after reduction and 66 kDa in its reduced and N-deglycosylated form. The polypeptide is capable of forming dimmers and bins to TGF-β and TGF-β receptors (see below). Endoglin may be phosphorylated. Preferably, Endoglin refers to human Endoglin. More preferably, human Endoglin has an amino acid sequence as shown in Genebank accession number AAC63386.1, GI: 3201489. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific Endoglin. Variants may be allelic variants, splice varaiants or any other species specific homologs, paralogs, or orthologs. Moreover, the variants referred to herein include fragments of the specific Endoglin or the aforementioned types of variants as long as these fragments have the essential immunological and biological properties as referred to above. Such fragments may be, e.g., degradation products of Endoglin. Further included are variants which differ due to posttranslational modifications such as phosphorylation or myristylation.

The term "soluble (s)Flt-1" as used herein refers to polypeptide which is a soluble form of the VEGF receptor FLT1. It was identified in conditioned culture medium of human umbilical vein endothelial cells. The endogenous soluble FLT1 (sFLT1) receptor is chromatographically and immunologically similar to recombinant human sFLT1 and binds [125I] VEGF with a comparable high affinity. Human sFLT1 is shown to form a VEGF-stabilized complex with the extracellular domain of KDR/Flk-1 in vitro. Preferably, sFLT1 refers to human sFLT1. More preferably, human sFLT1 can be deduced from the amino acid sequence of Flt-1 as shown in Genebank accession number P17948, GI: 125361. An amino acid sequence for mouse sFLT1 is shown in Genebank accession number BAA24499.1, GI: 2809071. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFLT1. Variants may be allelic variants, splice variants or any other species specific homologs, paralogs, or orthologs. Moreover, the variants referred to herein include fragments of the specific sFLT1 or the aforementioned types of variants as long as these fragments have the essential immunological and biological properties as referred to above. Such fragments may be, e.g., degradation products of sFLT1. Further included are variants which differ due to posttranslational modifications such as phosphorylation or myristylation.

Determining the amount of the peptides or polypeptides referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an mass to charge (m/z) variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.
First, binding of a ligand may be measured directly, e.g. by NMR or surface plasmon resonance.
Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured.
Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to identify the cause of a cardiac necrosis. Therefore, the reference amount is to be chosen so that either a difference or a similarity in the compared amounts allows identifying those subjects which have a certain angiogenic status, i.e. a pro-angiogenic status or an anti-angiogenic status.

Accordingly, the term "reference amounts" as used herein refers to amounts of the polypeptides which allow allocating the angiogenic status of a subject as either pro-angiogenic or anti-angiogenic. Therefore, the reference may either be derived from (i) a subject known to have a pro-angiogenic status or (ii) a subject known to have an anti-angiogenic status. Moreover, the reference amounts, preferably, define thresholds. Suitable reference amounts or threshold amounts may be determined by the method of the present invention from a reference sample to be analyzed together, i.e. simultaneously or subsequently, with the test sample. A preferred reference amount serving as a threshold may be derived from the upper limit of normal (ULN), i.e. the upper limit of the physiological amount to be found in a population of subjects (e.g. patients enrolled for a clinical trial). The ULN for a given population of subjects can be determined by various well known techniques. A suitable technique may be to determine the median of the population for the peptide or polypeptide amounts to be determined in the method of the present invention. The ULN for P1GF referred to herein, preferably, varies between 3 and 8 pg/ml. More preferably, the ULN for the said P1GF is 5 pg/ml. The ULN for Endoglin referred to herein, preferably, varies between 1 and 4 ng/ml. More preferably, the ULN for the said Endoglin is 3 ng/ml. The ULN for sFLT1 referred to herein, preferably, varies between 70 and 80 ng/ml. More preferably, the ULN for the said sFLT1 is 75 ng/ml.

In principle, it has been found that with respect to the ULN an increased amount of P1GF and sFLT1 and a decreased amount of Endoglin are indicative for an anti-angiogenic status whereas with respect to the ULN a decreased amount of PLGF and sFLT1 and an increased amount of Endoglin are indicative for a pro-angiogenic status. Thus, in a preferred embodiment of the method of the present invention, with respect to the ULN an increased amount of PLGF and sFLT1 and a decreased amount of Endoglin are indicative for an anti-angiogenic status. In another preferred embodiment of the method of the present invention, with respect to the ULN a decreased amount of PLGF and sFLT1 and an increased amount of Endoglin are indicative for a pro-angiogenic status.

Advantageously, it has been found in the study underlying the present invention that a combination of P1GF, Endoglin and sFLT1 as biomarkers are required to determine the angiogenic status of a subject in a reliable and efficient manner. Moreover, it has been found that each of said biomarkers is statistically independent from each other. Accordingly, the method of the present invention provides for a highly reliable diagnosis. As described above, the techniques which are currently used to resolve this issue are time consuming and cost intensive. The method of the present invention, however, allows a reliable, fast and less cost intensive diagnosis and can be implemented even in portable assays, such as test stripes. Therefore, the method is particularly well suited for diagnosing emergency patients. Thanks to the findings of the present invention, a suitable angiogenic therapy for a subject can be reliably selected. Severe side effects caused by the wrong treatment of patients can be avoided.

In a preferred embodiment of the method of the present invention, said method further comprises determining the amount of TGF-β or a variant thereof in step a) and comparing said amount with a reference amount in step b). Preferably, said reference amount for TGF-β is the ULN. More preferably, with respect to the ULN an increased amount of TGF-β is indicative for a pro-angiogenic status while a decreased amount is indicative for an anti-angiogenic status.

The term "TGF-β (Transforming Growth Factor-beta)" as used herein refers to TGF-β1 a member of the TGF-beta polypeptide family of structurally related protein growth factors. It has been originally identified as stimulus for anchorage-independent growth of fibroblasts. Various further activities are meanwhile well known in the art. TGF-β1 is involved in neovascularization and vessel wall integrity (Bertolino Chest (Supplement) 2005, 128:585S-590S). Preferably, TGF-β refers to human TGF-β. More preferably, human TGF-β comprises an amino acid sequence as shown in Genebank accession number AAB26284, GI: 299150. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFLT1. Variants may be allelic variants or any other species specific homologs, paralogs, or orthologs. Moreover, the variants referred to herein include fragments of the specific sFLT1 or the aforementioned types of variants as long as these fragments have the essential immunological and biological properties as referred to above. Such fragments may be, e.g., degradation products of sFLT1. Further included are variants which differ due to posttranslational modifications such as phosphorylation or myristylation.

The present invention also relates to a method of diagnosing whether a subject suffering from coronary heart disease is susceptible for a pro-angiogenic therapy comprising:
a) determining the amounts of PLGF or a variant thereof, Endoglin or a variant thereof and sFLT1 or a variant thereof in a sample of a subject suffering from coronary heart disease; and
b) comparing the amount determined in step a) with reference amounts, whereby it is to be diagnosed whether the subject is susceptible for a pro-angiogenic therapy.

The term "pro-angiogenic therapy" as recited above relates to a therapy which induces or enhances the process of angiogenesis systemically or topically in a subject. Preferably, said pro-angiogenic therapy comprises administration of an angiogenic drug, preferably, selected from the group consisting of: VEGF, P1GF, Endoglin, anti-Fit-1 antibodies and ALK5 modifiers.

Specifically, it has been found that with respect to the ULN an increased amount of PLGF and sFLT1 and a decreased amount of Endoglin are indicative for a subject having an anti-angiogenic status and, thus, being susceptible for a pro-angiogenic therapy.

In a more preferred embodiment of the aforementioned method of the present invention, with respect to the ULN an increased amount of PLGF and sFLT1 and a decreased amount of Endoglin are indicative for a subject being susceptible for a pro-angiogenic therapy.

In another preferred embodiment of the aforementioned method of the present invention, the method further comprises determining the amount of TGF-β or a variant thereof in step a) and comparing said amount with a reference amount. Preferably, the reference amount for TGF-β is the ULN. More preferably, with respect to the ULN an increased amount of TGF-β is indicative for a subject being susceptible for a pro-angiogenic therapy.

The present invention further encompasses a device for diagnosing the angiogenic status of subject suffering from coronary heart disease comprising:
a) means for determining the amount of PLGF or a variant thereof, Endoglin or a variant thereof and sFLT1 or a variant thereof; and
b) means for comparing the amounts determined by the means of a) with a reference amounts,
whereby the device is adapted for carrying out the method of the present invention referred to above.

The term "device" as used herein relates to a system of means comprising at least the aforementioned means operatively linked to each other as to allow the prediction. Preferred means for determining the amount of a one of the aforementioned polypeptides as well as means for carrying out the comparison are disclosed above in connection with the method of the invention. How to link the means in an operating manner will depend on the type of means included into the device. For example, where means for automatically determining the amount of the peptides are applied, the data obtained by said automatically operating means can be processed by, e.g., a computer program in order to obtain the desired results. Preferably, the means are comprised by a single device in such a case. Said device may accordingly include an analyzing unit for the measurement of the amount of the peptides or polypeptides in an applied sample and a computer unit for processing the resulting data for the evaluation. The computer unit, preferably, comprises a database including the stored reference amounts or values thereof recited elsewhere in this specification as well as a computer-implemented algorithm for carrying out a comparison of the determined amounts for the polypeptides with the stored reference amounts of the database. Computer-implemented as used herein refers to a computer-readable program code tangibly included into the computer unit. Alternatively, where means such as test stripes are used for determining the amount of the peptides or polypeptides, the means for comparison may comprise control stripes or tables allocating the determined amount to a reference amount. The test stripes are, preferably, coupled to a ligand which specifically binds to the peptides or polypeptides referred to herein. The strip or device, preferably, comprises means for detection of the binding of said peptides or polypeptides to the said ligand. Preferred means for detection are disclosed in connection with embodiments relating to the method of the invention above. In such a case, the means are operatively linked in that the user of the system brings together the result of the determination of the amount and the diagnostic or prognostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the natriuretic peptide, Plasmon surface resonace devices, NMR spectrometers, mass- spectrometers etc.) and/or evaluation units/devices referred to above in accordance with the method of the invention.

Also, the present invention relates to a device for diagnosing whether a subject is susceptible for a pro-angiogenic therapy comprising:
a) means for determining the amount of PLGF or a variant thereof, Endoglin or a variant thereof and sFLT1 or a variant thereof; and
b) means for comparing the amounts determined by the means of a) with a reference amounts,
whereby the device is adapted for carrying out the method of the present invention referred to above.

Moreover, the present invention relates to a kit adapted for carrying out the method of the present invention referred to above comprising:
a) means for determining the amount of PLGF or a variant thereof, Endoglin or a variant thereof and sFLT1 or a variant thereof; and
b) means for comparing the amounts determined by the means of a) with a reference amounts,
whereby the kit is adapted for carrying out the method of the present invention referred to above. Preferably, the kit comprises instructions for carrying out the said method of the present invention.

The term "kit" as used herein refers to a collection of the aforementioned means, preferably, provided in separately or within a single container. The container, also preferably, comprises instructions for carrying out the method of the present invention.

Finally, the present invention encompasses a kit adapted for carrying out the method of the present invention referred to above comprising:
a) means for determining the amount of PLGF or a variant thereof, Endoglin or a variant thereof and sFLT1 or a variant thereof; and
b) means for comparing the amounts determined by the means of a) with a reference amounts,
whereby the kit is adapted for carrying out the method of the present invention referred to above. Preferably, the kit comprises instructions for carrying out the said method of the present invention.

All references cited in this specification are herewith incorporated by reference with respect to their entire disclosure content and the disclosure content specifically mentioned in this specification.

The figure show:
**Figure 1****:** Linear regression analysis of sFLT1 and y Endoglin patients suffering from coronary heart disease.
**Figure 2****:** Linear regression analysis of sFLT1 and P1GF in patients suffering from coronary heart disease.
**Figure 3****:** Linear regression analysis of Endoglin and P1GF in patients suffering from coronary heart disease.

The following Examples shall merely illustrate the invention. They shall not be construed, whatsoever, to limit the scope of the invention.

### Example: P1GF, sFLT1 and Endoglin are statistically independent common predictors for the angiogenic status in patients suffering from coronary heart disease

A total of 274 patients suffering from coronary heart diseases were investigated for blood levels of sFLT1, Endoglin, P1GF and TGF-β1. The medical history of all patients was carefully recorded including potential risk factors for coronary heart diseases such as smoking, diabetes, arterial hypertension, or previous myocardial infarction.

Patients were analyzed by echocardiography. Moreover, the left ventricular ejection fraction (LVEF) was determined. Patients were divided into groups of LVEF of less than 40% (systolic dysfunction), 40-60% (ambiguous) and more than 60% (no systolic dysfunction). The patients having systolic dysfunction or being ambiguous were also investigated by coronary angiography. A coronary heart disease was confirmed if the vessels of the coronary system showed at least one stenosis resulting in 50% diminished volume. Patients were further subclassified according to one, two or three vessel diseases.

Blood levels of sFLT1, P1GF and Endoglin were determined using the commercially available Immunoassays "Quantikine" (Catalog numbers DVR100B, DPG00 and DNDG00) from R & D Systems, USA.

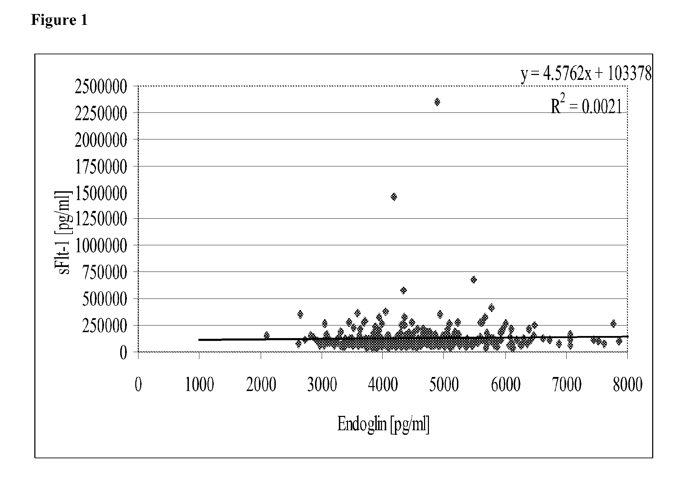

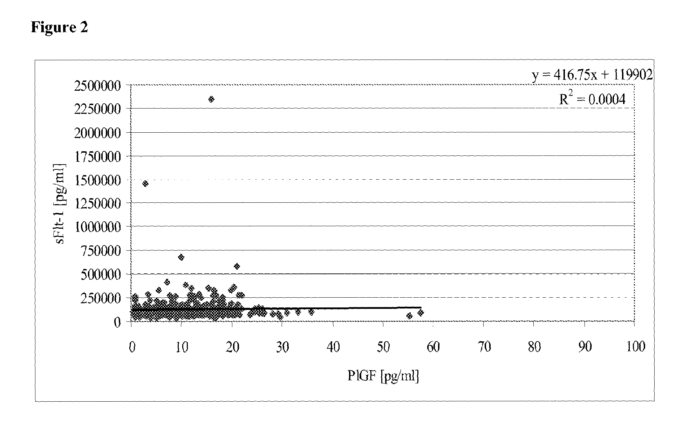

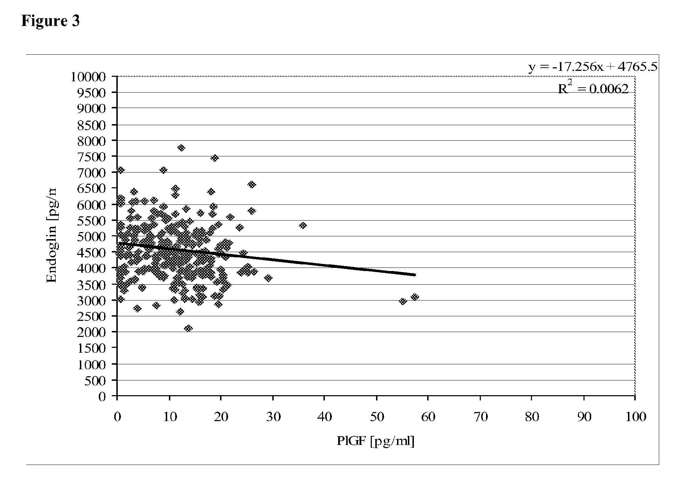

The results of the study are summarized in the following tables 1 to 4. Moreover, P1GF, Endoglin and SFLT1 are statistically independent from each other as shown by linear regression analysis (see Figures 1 to 3).

**Table 1: PIGF quartile in patients with documented coronary artery disease.**

| | **PIGF [pg/ml] N = 274** | | | |
|---|---|---|---|---|
| | Diagnose Group I: CAD | | | |
| | 1. Quartil | 2.Quartil | 3.Quartil | 4.Quartil |
| N | 62 | 63 | 76 | 73 |
| Median PIGF pg/ml | 2.17 | 8.45 | 12.75 | 18.49 |
| Range | 0.42-5.27 | 5.33-10.83 | 10.90-15.51 | 15.82-57.43 |
| Age, median | 66 | 65 | 66 | 69 |
| Male (n) | 41 | 41 | 54 | 45 |
| Female (n) | 21 | 22 | 22 | 28 |
| Height (m) median | 1.70 | 1.70 | 1.68 | 1.70 |
| Weight (kg) | 80.0 | 78.0 | 80.0 | 80.0 |
| LVEF (%) | | | | |
| > 60 % | 45 | 44 | 50 | 42 |
| 40-60% | 3 | 5 | 11 | 8 |
| < 40 % | 14 | 14 | 15 | 23 |
| | | | | |
| LA (mm), median | 38.5 | 38.0 | 41.0 | 41.0 |
| SEP (mm), median | 38.5 | 38.0 | 41.0 | 41.0 |
| Coronary artery disease | | | | |
| 1-vessel disease | 18 | 17 | 11 | 17 |
| 2-vessel disease | 15 | 15 | 22 | 16 |
| 3-vessel disease | 22 | 26 | 33 | 31 |
| | | | | |
| Smoker (n) | 27 | 32 | 39 | 37 |
| | | | | |
| Diabetes (n) | 18 | 15 | 23 | 28 |
| Art. Hypertension (n) | 52 | 41 | 52 | 47 |
| Heart Rate | 71 | 62 | 69 | 70 |
| Previous MI (n) | 23 | 22 | 26 | 30 |

**Table 2: Endoglin quartile in patients with documented coronary artery disease**

| | **Endoglin [ng/ml] N = 274** | | | |
|---|---|---|---|---|
| | Diagnose Group I: CAD | | | |
| | 1. Quartil | 2. Quartil | 3. Quartil | 4. Quartil |
| N | 72 | 72 | 65 | 65 |
| Median Endoglin ng/ml | 3.56 | 4.21 | 4.74 | 5.59 |
| Range | 2.11-3.88 | 3.88-4.43 | 4.43-5.07 | 5.09-27.9 |
| Age, median | 68 | 66 | 64 | 66 |
| Male (n) | 45 | 50 | 43 | 43 |
| Female (n) | 27 | 22 | 22 | 22 |
| Height (m) median | 1.70 | 1.70 | 1.68 | 1.69 |
| Weight (kg) | 80.0 | 80.0 | 80.0 | 78.0 |
| LVEF (%) | | | | |
| > 60 % | 40 | 48 | 49 | 44 |
| 40-60% | 10 | 5 | 5 | 7 |
| < 40 % | 22 | 19 | 11 | 14 |
| | | | | |
| LA (mm), median | 40.0 | 41.0 | 40.0 | 38.0 |
| SEP (mm), median | 12.5 | 12.0 | 12.0 | 12.0 |
| Coronary artery disease | | | | |
| 1-vessel disease | 17 | 17 | 16 | 13 |
| 2-vessel disease | 14 | 18 | 17 | 19 |
| 3-vessel disease | 32 | 28 | 27 | 25 |
| | | | | |
| Smoker (n) | 36 | 34 | 29 | 36 |
| | | | | |
| Diabetes (n) | 26 | 23 | 16 | 19 |
| Art. Hypertension (n) | 48 | 48 | 51 | 45 |
| Heart Rate | 65 | 69 | 70 | 68 |
| Previous MI (n) | 30 | 26 | 24 | 21 |

**Table 3: sFLT1 quartile in patients with documented coronary artery disease**

| | **sFLT1 [ng/ml] N = 274** | | | |
|---|---|---|---|---|
| | Diagnose Group I: CAD | | | |
| | 1. Quartil | 2. Quartil | 3. Quartil | 4. Quartil |
| N | 68 | 70 | 71 | 65 |
| Median sFlt-1 ng/ml | 66.1 | 82.3 | 107.4 | 173.2 |
| Range | 37.3-74.7 | 74.8-94.8 | 95.0-127.8 | 129.0-2343.7 |
| Age, median | 66 | 65.5 | 66 | 66 |
| Male (n) | 41 | 52 | 46 | 42 |
| Female (n) | 27 | 18 | 25 | 23 |
| Height (m) median | 1.68 | 1.70 | 1.70 | 1.70 |
| Weight (kg) | 77.8 | 84.0 | 77.0 | 78.0 |
| LVEF (%) | | | | |
| > 60 % | 53 | 38 | 53 | 37 |
| 40-60% | 11 | 12 | 4 | 7 |
| < 40 % | 4 | 20 | 14 | 21 |
| | | | | |
| LA (mm), median | 40.0 | 41.0 | 38.0 | 40.0 |
| SEP (mm), median | 13.0 | 12.5 | 11.0 | 12.0 |
| Coronary artery disease | | | | |
| 1-vessel disease | 16 | 18 | 14 | 15 |
| 2-vessel disease | 16 | 19 | 15 | 18 |
| 3-vessel disease | 27 | 29 | 32 | 24 |
| | | | | |
| Smoker (n) | 38 | 39 | 36 | 32 |
| | | | | |
| Diabetes (n) | 23 | 24 | 19 | 18 |
| Art. Hypertension (n) | 49 | 52 | 53 | 38 |
| Heart Rate | 69 | 66 | 70 | 67 |
| Previous MI (n) | 18 | 23 | 29 | 31 |

**Table 4: TGF-β1 quartile in patients with documented coronary artery disease**

| | **TGF-β1 [pg/ml] N =** | | | |
|---|---|---|---|---|
| | Diagnose Group I: CAD | | | |
| | 1. Quartil | 2. Quartil | 3. Quartil | 4. Quartil |
| N | 68 | 70 | 71 | 65 |
| Median TGF-β1 pg/ml Range | 13712 | 20936 | 26464 | 33676 |
| Age, median | 67.0 | 68.0 | 66.5 | 63.0 |
| Male (n) | 41 | 44 | 44 | 51 |
| Female (n) | 26 | 23 | 20 | 24 |
| Height (m) median | 1.69 | 1.70 | 1.70 | 1.70 |
| Weight (kg) | 77.8 | 80.0 | 82.0 | 80.0 |
| LVEF (%) | | | | |
| > 60 % | 61 | 68 | 63 | 54 |
| 40-60% | 9 | 14 | 9 | 10 |
| < 40 % | 24 | 13 | 23 | 28 |
| | | | | |
| LA (mm), median | 1.69 | 1.70 | 1.70 | 1.70 |
| SEP (mm), median | 77.8 | 80.0 | 82.0 | 80.0 |
| Coronary artery disease | | | | |
| 1-vessel disease | 12 | 16 | 14 | 20 |
| 2-vessel disease | 18 | 17 | 12 | 20 |
| 3-vessel disease | 26 | 27 | 30 | 29 |
| | | | | |
| Smoker (n) | 35 | 31 | 31 | 38 |
| | | | | |
| Diabetes (n) | 25 | 21 | 16 | 21 |
| Art. Hypertension (n) | 48 | 47 | 44 | 52 |
| Heart Rate | 67 | 66 | 70 | 68 |
| Previous MI (n) | 18 | 18 | 24 | 41 |

## Claims

1. A method for diagnosing the angiogenic status of a subject suffering from coronary heart disease comprising:
c) determining the amounts of PLGF or a variant thereof, Endoglin or a variant thereof and sFLT1 or a variant thereof in a sample of a subject suffering from coronary heart disease; and
d) comparing the amounts determined in step a) with reference amounts, whereby the angiogenic status is to be diagnosed.

2. The method of claim 1, wherein the reference amount is the ULN.

3. The method of claim 2, wherein with respect to the ULN an increased amount of PLGF and sFLT1 and a decreased amount of Endoglin are indicative for an anti-angiogenic status.

4. The method of claim 2, wherein with respect to the ULN a decreased amount of PLGF and sFLT1 and an increased amount of Endoglin are indicative for a pro-angiogenic status.

5. The method of any one of claims 1 to 4, wherein the method further comprises determining the amount of TGF-β or a variant thereof in step a) and comparing said amount with a reference amount in step b).

6. The method of claim 5, wherein the reference amount for TGF-β is the ULN.

7. The method of claim 6, wherein with respect to the ULN an increased amount of TGF-β is indicative for a pro-angiogenic status while a decreased amount is indicative for an anti-angiogenic status.

8. A method of diagnosing whether a subject suffering from coronary heart disease is susceptible for a pro-angiogenic therapy comprising:
c) determining the amounts of PLGF or a variant thereof, Endoglin or a variant thereof and sFLT1 or a variant thereof in a sample of a subject suffering from coronary heart disease; and
d) comparing the amount determined in step a) with reference amounts, whereby it is to be diagnosed whether the subject is susceptible for a pro-angiogenic therapy.

9. The method of claim 8, wherein the reference amount is the ULN.

10. The method of claim 9, wherein with respect to the ULN an increased amount of PLGF and sFLT1 and a decreased amount of Endoglin are indicative for a subject being susceptible for a pro-angiogenic therapy

11. The method of any one of claims 8 to 10, wherein the method further comprises determining the amount of TGF-β or a variant thereof in step a) and comparing said amount with a reference amount.

12. The method of claim 15, wherein the reference amount for TGF-β is the ULN.

13. The method of claim 12, wherein with respect to the ULN an increased amount of TGF-β is indicative for a subject being susceptible for a pro-angiogenic therapy.

14. The method of any one of claims 8 to 13, wherein said pro-angiogenic therapy comprises administration of an angiogenic drug.

15. The method of any one of claims 2 to 14, wherein the ULN for PLGF or a variant thereof is 5 pg/ml.

16. The method of any one of claims 2 to 15, wherein the ULN for sFLT1 or a variant thereof is 3 ng/ml.

17. The method of any one of claims 2 to 16, wherein the ULN for Endoglin or a variant thereof is 75 ng/ml.

18. The method of any one of claims 6, 7 or 11 to 17, wherein the ULN for TGF-β or a variant thereof is 20,000 pg/ml.

19. The method of any one of claims 1 to 18, wherein said subject is a human.

20. A device for diagnosing the angiogenic status of subject suffering from coronary heart disease comprising:
c) means for determining the amount of PLGF or a variant thereof, Endoglin or a variant thereof and sFLT1 or a variant thereof; and
d) means for comparing the amounts determined by the means of a) with a reference amounts,
whereby the device is adapted for carrying out the method of any one of claims 1 to 7.

21. A device for diagnosing whether a subject is susceptible for a pro-angiogenic therapy comprising:
c) means for determining the amount of PLGF or a variant thereof, Endoglin or a variant thereof and sFLT1 or a variant thereof; and
d) means for comparing the amounts determined by the means of a) with a reference amounts,
whereby the device is adapted for carrying out the method of any one of claims 8 to 19.

22. A kit adapted for carrying out the method of any one of claims 1 to 7 comprising:
a) means for determining the amount of PLGF or a variant thereof, Endoglin or a variant thereof and sFLT1 or a variant thereof; and
b) means for comparing the amounts determined by the means of a) with a reference amounts,
whereby the kit is adapted for carrying out the method of any one of claims 1 to 7.

23. The kit of claim 22, further comprising instructions for carrying out the method of any one of claims 1 to 7.

24. A kit adapted for a carrying out the method of any one of claims 8 to 19 comprising:
a) means for determining the amount of PLGF or a variant thereof, Endoglin or a variant thereof and sFLT1 or a variant thereof; and
b) means for comparing the amounts determined by the means of a) with a reference amounts,
whereby the kit is adapted for carrying out the method of any one of claims 8 to 19.

25. The kit of claim 24, wherein a kit for the comprising instructions for carrying out the method of any one of claims 8 to 19.
